# EUROPEAN PATENT APPLICATION

(11) **EP 4 826 369 A1**
(43) Date of publication of application: **21.10.2026**
(21) Application number: 25171320.2
(22) Date of filing: 17.04.2025
(51) Int. Cl.: G01N 27/12, G01N 27/22, G01N 27/414, G01N 33/00

(54) **SENSOR AND METHOD OF MANUFACTURE THEREOF**

(71) Applicant: Aalto University Foundation sr, 00076 Aalto (FI)
(72) Inventor: LYU, Zhongpeng, 00076 Aalto (FI); TAN, Hongwei, 00076 Aalto (FI); HOSSEINI SKOKOUH, Seyed, 00076 Aalto (FI); JÄÄNVIRTA, Antti, 00076 Aalto (FI); JIANG, Pan, 00076 Aalto (FI)
(74) Representative: Laine IP Oy

(57) **Abstract**

According to an example aspect of the present invention, there is provided a resistive sensor for sensing at least one gaseous substance, the resistive sensor comprising: at least one first electronic device for electrically sensing the at least one gaseous substance with a first sensitivity, said at least one first electronic device comprising a first composition of MXene and optionally lignin, and at least one second electronic device for electrically sensing the at least one gaseous substance with a second sensitivity, said at least one second electronic device comprising a second composition of MXene and optionally lignin, wherein the second composition is different from the first composition such that the second sensitivity differs from the first sensitivity.

## Description

### FIELD

The present disclosure relates to the field of sensing gaseous substances.

### BACKGROUND

Sensor types for sensing analytes, such as gaseous substances, include metal-oxide, MOx, sensors as well as electrochemical sensors. Such types of sensors may provide limited selectivity to different analytes and may exhibit complex designs comprising various different materials. As such, sensors such as those disclosed above may be difficult to manufacture. Moreover, such sensor types may be problematic to recycle, if at all, as the structure of such sensors may comprise a plurality of different materials. Moreover, such sensors are typically difficult to apply for a plurality of analytes. Therefore, there is a need for simplified devices and designs with relatively few components, having, for example, a small size, simple manufacturing scheme and that may further be designed with an expected environmentally friendly expiratory lifetime.

### SUMMARY OF THE INVENTION

The invention is defined by the features of the independent claims. Some specific embodiments are defined in the dependent claims.

According to a first aspect of the present invention, there is provided a sensor for sensing at least one gaseous substance, the sensor comprising: at least one first electronic device for electrically sensing the at least one gaseous substance with a first sensitivity, said at least one first electronic device comprising a first composition of MXene and optionally lignin; and at least one second electronic device for electrically sensing the at least one gaseous substance with a second sensitivity, said at least one second electronic device comprising a second composition of MXene and optionally lignin; wherein the second composition is different from the first composition such that the second sensitivity differs from the first sensitivity.

According to a second aspect of the present invention, there is provided a method comprising: obtaining at least one first electronic device comprising a first composition of MXene and optionally lignin, and at least one second electronic device comprising a second composition of MXene and optionally lignin, wherein the second composition is different from the first composition.

According to a third aspect of the present invention, there is provided a method comprising: measuring an electrical response from the plurality of electronic device comprised in the sensor; and based on the electrical response, deriving information on a presence of the at least one gaseous substance.

### BRIEF DESCRIPTION OF THE DRAWINGS

FIGs. 1A - 1E illustrate example compositions in accordance with at least some embodiments of the present disclosure; and
FIGs. 2A, 2B, 3A, 3B, 4A, 4B and 5 illustrate examples of apparatus, or parts thereof, in accordance with at least some embodiments of the present disclosure.

### EMBODIMENTS

"MXenes" are a group of inorganic compounds, the structures of which comprise atomically thin layers of transition metal carbides, transition metal nitrides and/or transition metal carbonitrides. Transition metals include titanium, Ti, vanadium, V, niobium, Nb, molybdenum, Mo, chromium, Cr, zirconium, Zr, manganese, Mn, technetium, Tc, and scandium, Sc. The group of MXenes include Ti₃C₂Tₓ, V₂CTₓ and Mo₂CTₓ, to name a few. As can be noted from the non-exhaustive list of MXenes above, MXenes comprise a functional termination Tₓ. The functional termination Tₓ may be, for example, oxygen O, fluorine F, hydroxyl OH or chlorine Cl, or a combination thereof.

Functional termination Tₓ may be a combination of groups, for example, a combination of a hydroxyl group, oxygen group and fluorine group, that is, Tₓ may be (OH)ₓ₁Oₓ₂Fₓ₃, wherein x1, x2 and x3 are the relative portions of said groups. Example of such an MXene is Ti₃C₂(OH)ₓ₁Oₓ₂Fₓ₃. The ratio of the hydroxyl, oxygen and fluorine groups, (that is, having x1, x2 and x3, respectively) may be different between two Ti₃C₂Tₓ, formulations in different electronic devices, for example. In other words, the proportion of said Tₓ groups may differ, although sum of said proportion should sum to x (of Tₓ).

MXene material may be manufactured with various processes, for example, by etching suitable precursors. MXene may be obtained by fluoride etching, electrochemical etching and/or water-free etching, for example. MXenes may be obtained from MAX phase of a precursor substance. MXenes may be manufactured by etching element "A" off from a corresponding "MAX" phase material. For example, etching aluminium, Al, (that is, an element A) off from a Ti₃AlC₂ precursor (that is, a MAX phase material) provides Ti₃C₂Tₓ (that is, an MXene).

MXenes may be characterized as having two-dimensional structures. In other words, thickness of such an MXene structure is substantially less than the other two dimensions. Such two-dimensional structures of MXenes may be termed "flakes". The terms "laminated" and "delaminated" in the context of the present disclosure refer to a property of an MXene to form layered or lamellar structures. Therefore, "delaminated" refers to disconnected or partially disconnected flakes of MXene. In manufacture of an MXene material such delaminated flakes may be dispersed in a solvent, for example. MXene structure may be at least in part characterized by said lamination (or delamination) and degree thereof. Moreover, the term "well-delaminated" refers to a characteristic that such a delamination property is obtained for a majority of MXenes structures within a material.

A type of MXene is determined by the functional termination (that is, Tₓ) and the Mₙ₊₁Xₙ layer (also known as "MX layer"). Example of an MX layer is thus, Ti₃C₂ or Mo₂C, for an MXene Ti₃C₂Tₓ or Mo₂CTₓ, respectively. An MXene is of different MXene type with respect to another MXene in terms of a difference in their functional terminations Tₓ, MX layer, or both functional terminations and the MX layers. A composition of MXene may comprise one or more than one different type of MXene. When MXene of a composition is said to be of a particular MXene type, such a composition comprises predominantly MXene of said MXene type, but may comprise different types of MXene at lower concentrations or ratios. For example, different MXene types having different functional terminations, may be present in the composition of MXene. In addition to MXene, a composition, such as a first composition and/or a second composition, may comprise lignin.

In accordance with the present disclosure, there is provided a sensor for sensing at least one gaseous substance said sensor comprising at least one first electronic device for electrically sensing the at least one gaseous substance with a first sensitivity and at least one second electronic device for electrically sensing the at least one gaseous substance with a second sensitivity. The at least one first electronic device comprises a first composition of MXene and optionally lignin. The at least one second electronic device comprises a second composition of MXene and optionally lignin. The second composition is different from the first composition such that the second sensitivity differs from the first sensitivity. Therefore, the difference between first sensitivity of the first electronic device and second sensitivity of the second electronic device is at least in part due to their compositions and differences therein. Moreover, said sensitivities are for at least one gaseous substance and concentrations thereof. The first and second sensitivities may be different for two or more gaseous substances, for example, for a first gaseous substance and a second gaseous substance. The sensor may comprise a substrate having said at least one first electronic device and/or said at least one second electronic device thereon. The sensor may comprise a plurality of electronic devices, such as more than two electronic devices.

In some embodiments, the first electronic device comprises a first composition of MXene and lignin. In some embodiments, the second electronic device comprises a second composition of MXene and lignin. A composition comprising lignin may be beneficial in that it may be used to obtain a degree of delamination of MXene in a composition of MXene and lignin of an electronic device, for example. The part of an electronic device having a composition comprising MXene and optionally lignin may also be known as a "sensing layer".

In an embodiment, the first composition and/or the second composition comprises MXene and optionally lignin, and further comprises at least one dopant, such as a noble metal, metal oxide, 2-dimensional material and/or a functional organic compound. Examples of a noble metal include copper, platinum and palladium. Examples of metal oxides include copper oxide, tin oxide and zinc oxide. 2-dimensional material, also known as single-layer material, refers to a crystalline solid material having a (substantially) single layer of atoms. Examples of 2-dimensional materials include graphene, Xenes, clays, transition metal dichalcogenides and 2-dimensional metal organic frameworks. Examples of functional organic compounds include metal-organic frameworks, ion-conjugate polymers and electrically conductive polymers. Such a dopant provides the benefit in that sensitivity of the first composition and/or the second composition is modified, such as increased.

In an embodiment, the at least one gaseous substance comprises at least one of: hydrogen H₂, ammonia NH₃, hydrogen sulfide H₂S, nitric oxide NO, nitric dioxide NO₂, carbon monoxide CO, or volatile organic compound (VOCs). VOCs include, for example, toluene C₆H₅CH₃, ethanol CH₃CH₂OH, methanol CH₃OH, methane CH₄, or acetone (CH₃)₂CO. Other gaseous substances may be applicable as well. A gaseous substance may also be known in some contexts as an "analyte". Moreover, ambient conditions may affect the electrical properties measurable from electronic devices of the sensor. These ambient conditions include humidity, temperature and air composition.

In the context of the present disclosure, the term "electronic device", also known as an "electronic component", refers to a discrete electronic element or physical entity part of an electronic system configured to affect electrons and/or their associated electromagnetic fields. An electronic device may comprise one or more electrical terminal via which said electronic device is electrically connectable to other electronic elements or entities. An electronic device may be passive or active. A passive electronic device may be a resistor or a capacitor, for example. An active electronic device may be a transistor, such as a field-effect transistor, FET, for example.

In the context of the present disclosure, the term "resistivity" is to be understood as electrical resistivity, often described in unit ohm meter, Ω ·m. Such a feature is characteristic for a material, and may be indirectly measured using conductance and/or resistance measurements for a certain geometry of said material. Moreover, "resistance" is to be understood as electrical resistance often described in ohms, Ω. Sometimes, conductivity and conductance may be used, i.e., the inverse of resistivity and resistance, respectively. Terms such as "resistivity of an electronic device", and statements alike, refer to the resistivity of the electronic device in a possible combination of the effects caused by the at least one gaseous substance in conjunction with said pattern and interaction therewith.

In the context of the present disclosure, "capacitance" refers to the capability of a component, material or the like, to store electrical charge often described in unit Farads, F, or coulombs per volt, C/V. Thus, a change in charge or voltage in such a component implies a change in capacitance. Further, a change in charge may be measured as a change in voltage via capacitance. Capacitance comprises at least two electrodes separated by a medium. Such a medium may be, for example, a dielectric material. In a capacitor, two electrodes are connected to one another via a displacement current in a gap, such as the dielectric medium, between said electrodes thus, separating said electrodes. The change in the electrical properties of one or both of the electrodes affects the capacitance of the capacitor.

A field-effect transistor, FET, comprises a semiconducting material, a source terminal and a drain terminal in contact with said semiconducting material. At least part of the semiconducting material, may also be known as an active layer or a channel. The channel is situated, thus, between and in contact with, the source terminal and the drain terminal. An FET also comprises a gate terminal. The gate terminal is separated from the channel by a dielectric such as an oxide layer, for example, silicon dioxide, SiO₂. Moreover, the conductivity of the channel is a function of the potential, or voltage, applied across the gate terminal and the source terminal. The gate terminal may be configured to modulate the electrical conductivity of the channel, based on voltage. A composition of MXene and optionally lignin may be characterized as a semiconducting material although its behavior might not necessarily *per se* be related to mechanisms typically attributed to semiconductor materials (that is, semiconductors), such as silicon. The semiconducting (i.e., semiconductive like) behavior of a composition of MXene may depend on inclusion of an ion-conjugate polymer, quaternary ammonium cation, and the like. A dielectric intercalant may lead to a semiconducting transport of the composition. Dielectric intercalants include, for example, polymers, organic molecules, and the like. Electrical conductivity of a composition of MXene and optionally lignin may depend on a dopant, and amount thereof. Electron transport may be thus, be altered based on the composition. Therefore, at least such an MXene material may be utilized as a semiconducting material.

The term "sensitivity" in the context of the present disclosure is to be understood as a capability of an electronic device and/or the sensor to detect the changes in an environment, and presence of at least one gaseous substance (or lack thereof), of said electronic device and/or sensor. In some embodiments, "sensitivity" describes the capability of an electronic device to experience a measurable electrical property, such as a resistance value and/or resistivity that is proportional to a concentration of a gaseous substance introduced on the electronic device. In other words, based on the composition of MXene and optionally lignin of an electronic device, the electrical property measured from said electronic device of the sensor is different than an electrical response measured from another electronic device of the sensor having a different composition of MXene and optionally lignin. For example, the range, the limit of detection, LOD, and/or limit of quantification LOQ, may be different, based on the composition of MXene and optionally lignin. Two or more electronic devices of the sensor may thus, have different sensitivities for the at least one gaseous substance. In addition, an electronic device may have different sensitivities for two or more gaseous substances. The term "sensing", or "electrically sensing", is to be understood as a measurable electrical response of an electronic device to an at least one gaseous substance, such as the measurement, quantification, and/or detection of the at least one gaseous substance. That is, a measurable quantity or change in the electrical properties, such as current, voltage, resistance and/or capacitance with respect to the at least one gaseous substance may be electrically sensed. The first composition and second composition are therefore different such that a first electronic device comprising the first composition differs from a second electronic device comprising the second composition, such that the difference is in terms of sensitivity to the at least one gaseous substance.

Sensitivity of an electronic device and differences in sensitivities of two or more electronic devices may be based on measured electrical response with respect to the at least one gaseous substance. The measured electrical response may be obtained using instruments capable of measuring electrical response and changes therein. For example, resistivity and/or resistance values may be measured across an electronic device of the sensor. The resistance values measured across an electronic device and/or resistivity computable thereof, may change based on at least the presence of the at least one gaseous substance and composition of said electronic device. Therefore, a change, such as the change in resistance, is based on the composition of MXene and optionally lignin within an electronic device in relation to the presence of at least one gaseous substance (or lack thereof). In some embodiments, the electrical response across an electronic device is measured continuously. In some embodiments, the resistance is measured concurrently across each electronic device of the sensor, such as resistive patterns of a resistive sensor. In an embodiment, the electrical responses of the plurality of electronic devices are measured concurrently.

In an embodiment, a sensor is configured to sense two or more gaseous substances. Additionally, such two or more gaseous substances may be sensed at different time points and/or concurrently. Therefore, the sensitivity of the at least two electronic devices may be different such that said electronic devices provide electrical responses different from one another. It is noted that differences in composition of MXene and optionally lignin as well as sensitivity based thereon, are applicable for embodiments, wherein three or more electronic devices are comprised in the sensor.

The sensitivity of an electronic device of a plurality of electronic devices is different from other electronic devices of the plurality of electronic devices based on at least a difference in composition of MXene and optionally lignin of said electronic devices. One benefit of such a sensor is that the plurality of electronic devices, arranged to provide different sensitivities and responses to the at least one gaseous substance, are used to electrically sense the at least one gaseous substance in combination with one another. An electrical response across an electronic device may be, for example, information on resistivity of a resistive pattern, such as resistance values or changes therein. An electrical response across an electronic device may be, for example, information on capacitance of a capacitive pattern. An electrical response across an electronic device may be, for example, information on gate terminal voltage of a field-effect transistor, FET. In some embodiments, the sensor is configured to sense a plurality of gaseous substances, or a mixture of gaseous substances. As such according to at least some embodiments a structurally simple and easily manufacturable sensor is provided.

In some embodiments, a plurality of same and/or similar electronic devices are comprised in the sensor so as to provide an improved signal-to-noise ratio via concurrent measurements and average thereof, for example.

In some embodiments, the second composition is different from the first composition in terms of MXene and/or lignin. Inclusion of lignin within a composition comprised in an electronic device provides some advantages and benefits. For example, for an electronic device having a composition of MXene and lignin, lignin may be arranged so as to separate MXene flakes in a structure from other MXene flakes, thereby modifying the composition and sensitivity of such an electronic device to the at least one gaseous substance. In other words, a difference in the ratio of the amount of lignin relative to the amount of MXene of an electronic device may result in a difference in the spacing between MXene flakes of a composition. For example, increased lignin concentration may increase spacing between MXene flakes. The degree of delamination of MXene flakes may be modified with the relative concentration of lignin of an electronic device of the sensor. The ratio of the amount of lignin relative to the amount of MXene may also affect electron transport of the composition.

In an embodiment, the second composition is different from the first composition in terms of at least one of: MXene; lignin; the ratio of the amount of lignin relative to the amount of MXene; or dopant. In some embodiments wherein the difference between the first composition and the second composition of two electronic devices is at least due to lignin, the difference may be because of difference in chemical composition of lignin between the compositions of said electronic devices. In some embodiments wherein the difference between the first composition and the second composition is at least due to MXene, said difference may be because of differences in type of MXene, MXene structure and/or MXene distribution between said electronic devices.

The difference in dopant means that the dopant atom or substance is different or that the concentration of the dopant differs an essential amount. The dopant may also refer to a combination of more than one dopants. The dopant may be, for example, a noble metal or a functional organic compound.

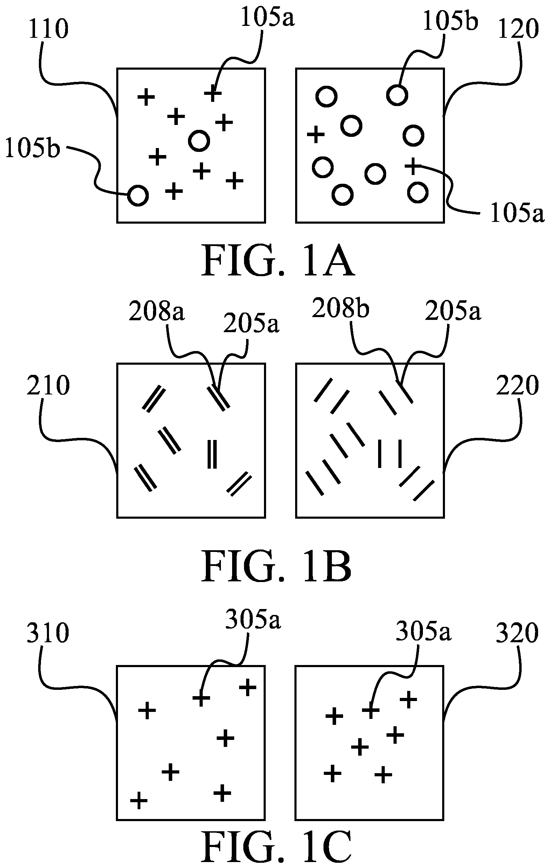

In an embodiment, the difference in sensitivity of at least two electronic devices for electrically sensing the at least one gaseous substance is due to a difference in at least one of: types of MXene; MXene structures; chemical compositions of lignin; ratios of the amount of lignin relative to MXene; MXene distributions; or dopant. A more detailed description and examples of items listed above are provided in the succeeding paragraphs. One or more of the above-listed differences may be applicable for a sensor, and/or for electronic devices therein. FIGs. 1A - 1E illustrate examples regarding differences between two compositions in two different electronic devices.

Regarding types of MXene, various MXene types are available. Examples of MXene types include Ti₃C₂Tₓ and Mo₂CTₓ. In some embodiments, an MXene for at least one electronic device has a formula Ti₃C₂Tₓ wherein Ti is titanium, C is carbon and Tₓ is a functional group. Tₓ may be, for example, an oxygen O group, a hydroxyl OH group, and/or a fluorine F group. The MXene type also refers to a difference in combination of compounds within the functional group Tₓ. The combination of O, OH and F in Tₓ may, thus, have a ratio or relative proportion to one another. For example, two Ti₃C₂Tₓ type MXenes may be different in terms of the functional group. For example, Ti₃C₂(Cl)ₓ and Ti₃C₂(OH)ₓ are examples of two MXene types, x is a positive integer. The numerical value for x need not be the same between the formulae. Ti₃C₂Tₓ type MXenes are beneficial in that Ti₃C₂Tₓ with different ratios of oxygen, fluorine and hydroxyl groups may be readily available and manufacturable from suitable precursors. MXene types of a composition may be quantified, for example, using nuclear magnetic resonance, NMR, spectroscopy.

MXene types may be different in terms of their combination of elemental groups in the functional group Tₓ of an MXene. Example of an MXene type is Ti₃C₂(OH)ₓ₁Oₓ₂Fₓ₃ such that x1, x2 and x3 are integers such that x1 is greater than x2 and x2 is greater than x3 and another example of an MXene type is Ti₃C₂(OH)_{y1}O_{y2}F_{y3} such that y1, y2 and y3 are integers such that y1 is less than y2, and y2 is less than y3. In an embodiment, the first composition of the at least one first electronic device comprises MXene having a formula Ti₃C₂(OH)ₓ₁Oₓ₂Fₓ₃ and the second composition of the at least one second electronic device comprises MXene having a formula Ti₃C₂(OH)_{y1}O_{y2}F_{y3} wherein at least one of x1, x2 or x3 is different from y1, y2 or y3, respectively. In an embodiment, MXene of a first electronic device comprises V₂CTₓ and MXene of a second electronic device comprises Mo₂CTₓ. As another example, a sensing layer of an electronic device may comprise an O or OH rich composition of Ti₃C₂Tₓ, and a sensing layer of another electronic device may comprise an F rich composition of Ti₃C₂Tₓ. FIG. 1A illustrates schematically two sensing layers of two electronic devices 110 & 120 having predominantly different MXene types therein, 105a & 105b, respectively. In other words, in FIG. 1A, plus marks "+" illustrate a first MXene type and circle marks "o" illustrate a second MXene type. As can be appreciated from FIG. 1A, small amounts of another MXene type, or plurality thereof, may be present in an electronic device comprising predominantly an MXene type.

Regarding MXene structure, although MXene type may be same or similar in the composition of the at least two electronic devices, the structure of MXene of the at least two electronic devices may differ. For example, MXene may be formed from varying degrees of delamination or lamination within a sensing layer of an electronic device, and as such the structure of MXene "flakes" may be different. FIG. 1B schematically illustrates two sensing layers of two electronic devices 210 & 220 having same MXene type 205a, but different MXene structures 208a & 208b, respectively. In FIG. 1B, MXene structure of sensing layer 210 comprises MXene 205a, said MXene 205a being more laminated than that of MXene 205a of the sensing layer of the other electronic device 220.

Lignin refers to a collection of organic macromolecules or organic polymers typically derivable from plants. Lignin may be graded based on the ratio and/or occurrence of lignin types (also known as lignin units) within said lignin. Lignin may have at least lignin types p-hydroxyphenyl (H), guaiacyl, and syringyl, typically termed H, G and S-units, respectively. Therefore, chemical composition of lignin refers to the contents and ratio of lignin types within a composition comprising lignin. Lignin may be graded based on lignin molecular structure and lignin composition. Such grading may therefore describe a unit of lignin (or plurality thereof) predominantly or primarily present within a material comprising lignin. Chemical composition of lignin may also be presented as a ratio of the lignin types, for example as an H:G:S ratio. The ratio between H, G and S units may be different with respect to wherefrom lignin has been derived. For example, lignin originating from hardwood may comprise predominantly G and S units, while lignin originating from softwood may comprise predominantly G units, and lignin originating herbaceous plant may comprise H, G and S units of lignin. Ratio of units in lignin material may be quantified using thioacidolysis, for example, in combination with gas and liquid chromatography-mass spectrometry, LC-MS and GC-MS.

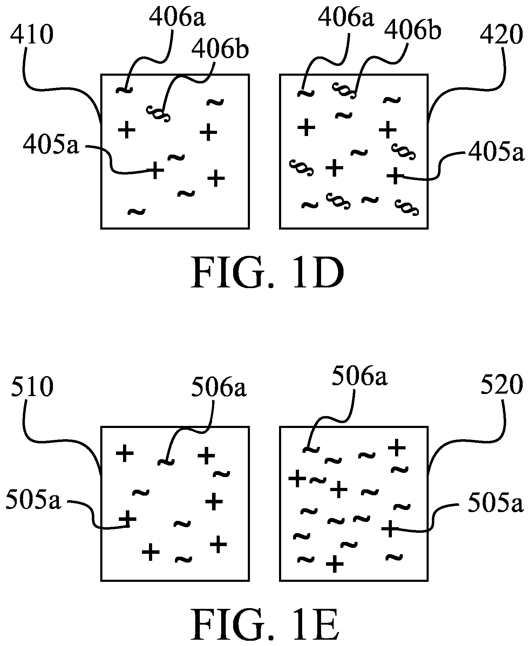

Regarding chemical composition of lignin, lignin may comprise different lignin types, such as S-units, H-units and G-units at different concentrations or ratios to one another, for example. Therefore, in some embodiments, a difference in sensitivity of the two or more electronic devices for the at least one gaseous substance is based on a difference in the occurrence and/or ratio of lignin types, such as S-units, H-units and G-units. In other words, the ratio of the lignin types may configure an electronic device to have a different sensitivity for the at least one gaseous substance compared to another electronic device. Therefore, a benefit of having different chemical compositions of lignin between two or more electronic devices is that such differences may provide different sensitivities and/or responses to the at least one gaseous substance. FIG. 1D, illustrates a first chemical composition of lignin having predominantly a first lignin type 406a and second chemical composition of lignin having predominantly a second lignin type 406b within compositions of sensing layers of the electronic devices 410 & 420. The sensing layers of electronic devices 410 & 420 comprise lignin and MXene 405a.

Although the abovementioned lignin types, that is, S-units, H-units and G-units of lignin are disclosed, and are acknowledged functional units for lignin, other functional groups or units may be available and/or observable for lignin. As such, the chemical composition of lignin depends on the lignin types available. For a lignin to comprise "predominantly" of a unit, said lignin is understood to comprise more of said unit than the other units in terms of volume, molecular mass and/or weight, for example. Thus, such a predominant unit may be considered a main or prevalent unit of lignin.

Regarding the ratio of the amount of lignin relative to MXene, in some embodiments the ratio of the amount of lignin relative to MXene is different between the at least two electronic devices, such as the first electronic device and the second electronic device. The first composition or the second composition may comprise a dopant. In an embodiment, the first composition is from 50 to 70 wt.% MXene and from 30 to 50 wt.% lignin. The first composition may comprise a dopant. In an embodiment, the first composition is from 40 to 60 wt.% MXene, from 20 to 30 wt.% lignin. For example, a first composition may comprise 70 wt.% MXene, and 30 wt.% and a second composition may comprise 50 wt.% MXene and 50 wt.% lignin. In an embodiment, a first composition of a first electronic device is 100 wt.% MXene, and 0 wt.% lignin and a second composition of a second electronic device is 75 wt.% MXene and 25 wt.% lignin. Regarding such ratios, weight-percent wt.%, is to be understood as the ratio of the mass of MXene with respect to the mass of lignin. In some embodiments, said ratio is relative to the dry-mass or dry-weight of the sensing layer.

In some embodiments, the at least two electronic devices have respective compositions of MXene and lignin, said compositions having at least different amount of lignin relative to MXene as a substantially lamellar structure of lignin and MXene layers. The sensing layers of the at least two electronic devices may have a different interlayer spacing of MXene flakes. FIG. 1E illustrates ratios of MXene 505a and lignin 506a within sensing layers of electronic devices 510 & 520. As can be appreciated, illustrated composition of the sensing layer of the electronic device 510 has less lignin 506a than composition of the sensing layer of the electronic device 520.

Regarding MXene distributions, although MXene type may be same or similar between the composition of sensing layers of the at least two electronic devices, the distribution of MXene in sensing layers of two or more electronic devices may differ. In other words, the composition of the MXene and optionally lignin may be different in terms of MXene distribution in the material. Such a difference may be obtained, for example, during manufacturing of MXene flakes, wherein different solvents may be used to obtain different MXene distributions for different electronic devices. Examples of solvents include water (H₂O) and organic solvents. Organic solvents include dimethyl sulfoxide (DMSO), N-methylpyrrolidone (NMP), N,N-dimethylacetamide (DMAc), N,N-dimethylformamide (DMF), and ethanol (EtOH), for example. As such, a difference in solvents used in manufacture of an ink and/or an electronic device, may at least in part provide a difference between the compositions of the electronic devices in terms of MXene distribution. In other words, in at least some embodiments, two or more electronic devices have compositions with different distributions of MXene in terms of placement and relative orientation of MXene flakes. FIG. 1C illustrates MXene distributions in compositions of electronic devices 310 & 320. As can be appreciated MXene 305a in the sensing layer of the electronic device 310 are more sparsely distributed than MXene 305a of the sensing layer of the electronic device 320. In at least some embodiments, the distribution of the MXene is not necessarily homogenous or uniform within an electronic device or a sensing layer but instead MXene in the electronic device or sensing layer may have different distribution of MXene in different spatial directions. For example, the distribution of MXene may vary in the thickness direction of the sensing layer.

In at least some embodiments, other substances, compounds and/or formulations than merely MXene, optionally lignin and dopant may be comprised in a sensing layer of an electronic device, that is, in a part of an electronic device wherein composition of MXene and optionally lignin resides. Such other substances, compounds and/or formulations may improve, for example, viscosity of ink from which an electronic device, or part thereof such as a pattern, is manufactured and/or to provide different mechanical properties for said ink and/or pattern of an electronic device, for example.

The electronic devices may be on a substrate. In some embodiments, the substrate is a biodegradable material, such as a paper-based material or polylactic acid - polyhydroxy butyrate, PLA-PHB, material. A benefit of such materials as a substrate is that biodegradability, or a degree thereof, may be possible. In some embodiments, glossy photo paper, or office paper is the substrate. In some embodiments, the substrate comprises a synthetic material. The synthetic material may comprise, for example, polyimide, polyethylene terephthalate, PTFE, polyethylene, PE, and/or polypropylene, PP. A benefit of such a synthetic material may be, for example, a durable and/or easily manufacturable sensor. It is a benefit of at least some embodiments that the sensor may be configured to operate in different scenarios having potentially different needs for material integrity of the substrate (for example, biodegradability, or mechanical durability).

A substrate may affect the measured electrical response, and therefore sensing of the at least one gaseous substance. Such may be the case if the sensing layer of the electronic device is on the substrate, for example, in an embodiment, wherein an electronic device comprises a resistive pattern or a capacitive pattern. As such calibration of measurable values may depend on the substrate, for example. The substrate may comprise, or be, a dielectric material.

In some embodiments, the substrate is a porous substrate, such as a cloth and/or fabric material, for example a woven or non-woven material. In some embodiments, the electronic devices are arranged on a porous or mesh-like substrate may be in contact with the at least one gaseous substance via and/or through such porous structure of the substrate. A benefit of a porous or mesh-like substrate is at least that such a sensor may be attachable to an entrance, or a recess through or past which the at least gaseous substance may flow, for example. For example, at least such a sensor may be installable to a ventilation pipe so as to measure the at least one gaseous substance propagating along such a pipe. A porous or mesh-like substrate may also protect the electronic devices and/or sensing layer therein from direct mechanical contact, for example, with debris, as, for example, the electronic devices may be situated on a surface opposite to that of said mechanical contact.

In some embodiments, the substrate is a singular object, such as a sheet of material. In some embodiments, the substrate may be a plurality of sheets. In other words, a substrate may comprise separate sheets of material, for example. In some embodiments, the substrate is flexible and/or foldable. For example, in some embodiments, a substrate is a sheet which may be folded and/or rolled. In some such embodiments, electrical response and changes therein regarding such folding and/or rolling should be taken into account via calibration, for example.

In some embodiments, the plurality of electronic devices is arranged on the substrate as an array, such as a grid of electronic devices. The plurality of electronic devices may be defined in terms of sensitivity to the at least one gaseous substance based on the composition of MXene and optionally lignin. In some embodiments, the sensing layer may have a geometric shape such that the surface area with respect to the outer dimension of an electronic device, or part thereof, may be optimized.

In some embodiments, the part of the electronic device comprising MXene and optionally lignin (that is, a pattern or a sensing layer), has a thickness from 50 nm, nanometers to 5 µm, micrometers. In some embodiments a pattern or a sensing layer has a thickness from 0.1 µm to 2 µm, such as from 0.5 µm to 1 µm. Such a thickness of an electronic device may be defined along a surface normal of the corresponding substrate to which said electronic device is provided. In an embodiment, wherein a gate terminal comprises a composition of MXene and optionally lignin, the thickness of the gate terminal (i.e., sensing layer), is from 50 nm to 100 nm, nanometers.

In some embodiments, comprising a printable sensing layer or a pattern of an electronic device, such as a first electronic device, may comprise a printed line having a width of 10 µm to 1000 µm, or 20 µm to 500 µm, and/or a length of 100 µm to 30 cm, 1 cm to 10 cm, 1 cm to 5 cm, 100 µm to 3000 µm, or 200 µm to 1000 µm. Such a line may be, for example, printed on the substrate using a suitable printing technique. In some embodiments, at least one electronic device, such as the first electronic device or the second electronic device, or sensing layer comprising the composition of MXene and optionally lignin, has a surface area from 0.1 mm² to 1500 mm², such as from 25 mm² to 500 mm², square millimeters.

In an embodiment, at least one electronic device is a resistor comprising a resistive pattern comprising a composition of MXene and optionally lignin. Such at least one electronic device may be a first electronic device and/or a second electronic device, for example. In an embodiment, at least one first electronic device is a resistor comprising a first resistive pattern comprising the first composition of MXene and optionally lignin and at least one second electronic device is a resistor comprising a second resistive pattern comprising the second composition of MXene and optionally lignin.

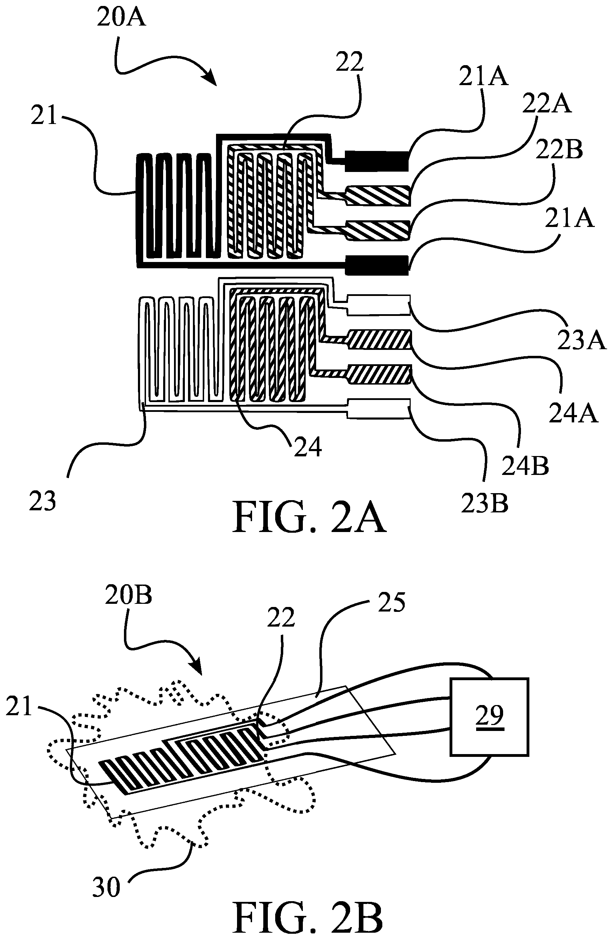

FIG. 2A illustrates a sensor 20A according to an embodiment. In FIG. 2A, the sensor 20A is a resistive sensor comprising four electronic devices 21, 22, 23 and 24. The four electronic devices are also four sensing layers. The four sensing layers are different from one another in terms composition and thus, in terms of sensitivity for sensing the at least one gaseous substance. For a resistor 21 - 24, a sensing layer may also be referred to as a resistive pattern. Moreover, the patterns 21 - 24 are connectable to other equipment, such as a measurement device configured for measuring resistance across said patterns 21 - 24, for example. As can be appreciated, the patterns may comprise contact areas 21A, 21B, 22A, 22B, 23A, 23B, 24A and 24B, such as contact pads. With at least such contact areas, a pattern may be attachable to additional electrical components, such as a measurement device configured to measure resistance and changes thereof across said resistive pattern(s).

FIG. 2B illustrates a sensor 20B according to an embodiment in the presence of a gaseous substance 30. In FIG. 2B, the sensor 20B is a resistive sensor. The resistive sensor comprises a substrate 25 and two resistive patterns 21, 22. As such, the resistive sensor 20B comprises a plurality of patterns, comprising a first pattern 21 having a first composition and a second pattern 22 having a second composition. The first composition comprises MXene and the second composition comprises MXene. The first composition may comprise lignin. The second composition may comprise lignin. The second composition of the second pattern 22 is different from the first composition of the first pattern 21 such that the second sensitivity differs from the first sensitivity. The patterns 21,22 are configured to be connected to measuring equipment 49, configured to measure resistance across said patterns 21,22, respectively. FIG. 2B also illustrates at least one gaseous substance 30, because of which a measurable change in resistance of the patterns 21,22 of the resistive sensor 20B may be observed. In other words, information, for example, on the presence or concentration of such at least one gaseous substance 30 may be derived. The resistance measured by the measuring equipment 49 may then be used to compute, for example correlated to, a concentration and/or composition of the at least one gaseous substance.

In an embodiment, at least one electronic device is a capacitor comprising two electrodes, at least one of said two electrodes comprising a composition of MXene and optionally lignin. The two electrodes the capacitor are compositionally different from one another. Such at least one electronic device may be, for example, the at least one first electronic device and/or second electronic device. In an embodiment, wherein one of the electrodes comprises MXene and optionally lignin, the other electrode comprises copper, Cu, and/or silver, Ag. The sensor may comprise a first electronic device and a second electronic device, such that the at least one first electronic device is a capacitor, and the at least one second electronic device is another capacitor. The capacitor, such as the capacitor being the first or second electronic device, comprises two electrodes, at least one of which comprises a composition of MXene and optionally lignin. The electrodes of a capacitor may be patterns, such as patterns that have been printable with a suitable ink.

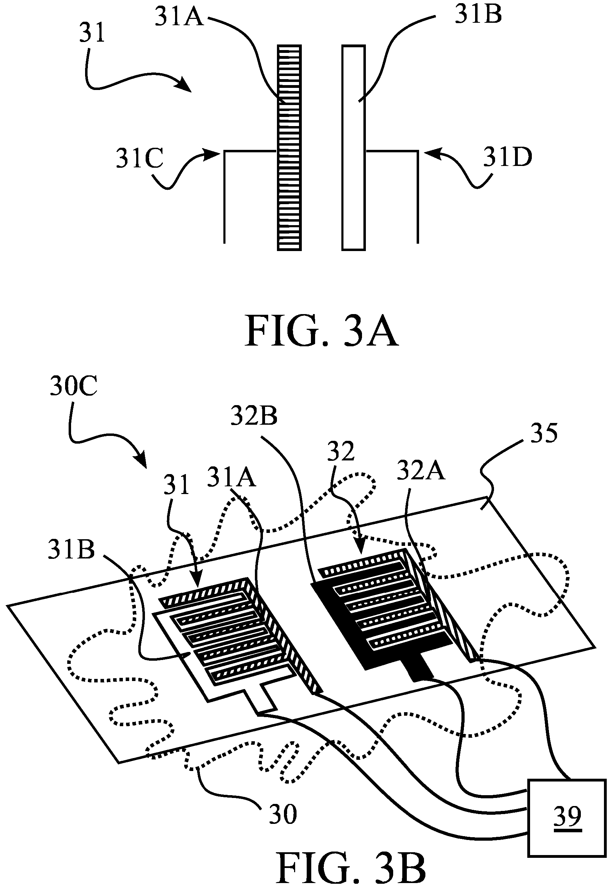

FIG. 3A illustrates an electronic device according to an embodiment. In FIG. 3A, the electronic device 31 is a capacitor. The electronic device 31 comprises two electrodes 31A and 31B and leads 31C, 31D connected thereto, respectively. One of the two electrodes 31A, 31B comprises a composition of MXene and optionally lignin. In other words, one of the electrodes is a sensing layer. The capacitor comprising the two electrodes may be referred to as a capacitive pattern. Based on the interaction between at least one gaseous substance and the electronic device 31, the electrical properties of the electronic device 31 is configured to change. This change in properties provides a measurable difference, for example, in voltage, different to that with a concentration of the at least one gaseous substance, such as in the absence of the at least one gaseous substance.

FIG. 3B illustrates a sensor according to an embodiment and, as a dashed line, at least part of at least one gaseous substance 30. In FIG. 3B, the sensor 30C is a capacitive sensor. The sensor 30C comprises two electronic devices 31,32 separate from one another. Pairs of electrodes 31A,31B and 32A,32B are arranged to form the two separate capacitors, that is electronic devices 31 and 32, respectively. The capacitors 31,32 of FIG. 3B may be termed as interdigital capacitive patterns. One of the two electrodes of an electronic device 31,32 comprises a composition of MXene and optionally lignin. Moreover, two electrodes of an electronic device 31,32 may have a composition of MXene and optionally lignin.

In an embodiment, a capacitor (that is, a capacitive electronic device) has a layered structure comprising three or more layers. In an embodiment, a capacitor comprises a first layer as a first electrode of the capacitor and a second layer as a second electrode of the capacitor, said first layer and second layer being separated by a gap. Such a gap may comprise a layer of dielectric material. In such a capacitor, the first layer or the second layer comprises the composition of MXene and optionally lignin, and the second layer or the first layer comprises an electrically conductive material, such as metal, respectively. The layer structure may be obtained by sequentially printing said layers on top of one another using suitable ink formulations, for example. Alternatively, the layers may be obtained on one or more substrates or sheets, and said obtained layers may be placed on top of each other such that said first and second layers are separated by a dielectric. Such embodiment may be beneficial at least in cases where a porous substrate is used, as the sensing layer (one of the two electrodes having the composition of MXene and optionally lignin) may be exposable to the at least one gaseous substance via said porous substrate. In an embodiment, the substrate on which the two electrodes are attached is the layer of dielectric material, and electrodes are on opposite surfaces thereof.

The sensor may comprise one or more electronic devices wherein one or more electronic device is a field-effect transistor, FET. In an embodiment, at least one electronic device is a field-effect transistor, FET, having a source terminal, a drain terminal, a gate terminal, and a semiconducting channel, said FET comprising a composition of MXene and optionally lignin. In an embodiment, the channel comprises the composition. In another embodiment, the gate terminal comprises the composition. In an embodiment, at least one first electronic device is a field-effect transistor, FET, comprising the first composition of MXene and optionally lignin and at least one second electronic device is an FET comprising the second composition of MXene and optionally lignin.

In an embodiment, a gate terminal of a FET electronic device comprises the composition of MXene and optionally lignin. That is, the gate terminal is a sensing layer of the FET electronic device. Based on interaction with at least one gaseous substance, the electrical response, and changes therein may be measured. In an embodiment, the gate terminal voltage, that is, the voltage of the gate with respect to the source or drain terminal, is configured to be measured. The sensing layer of a FET electronic device is arranged such that the sensing layer may be in contact with the at least one gaseous substance. The interaction may amend the work function of the gate terminal and thereby the threshold voltage of the FET electronic device. In an embodiment, the gate terminal of the at least one first FET comprises the first composition. In an embodiment, the gate terminal of the at least one second FET comprises the second composition.

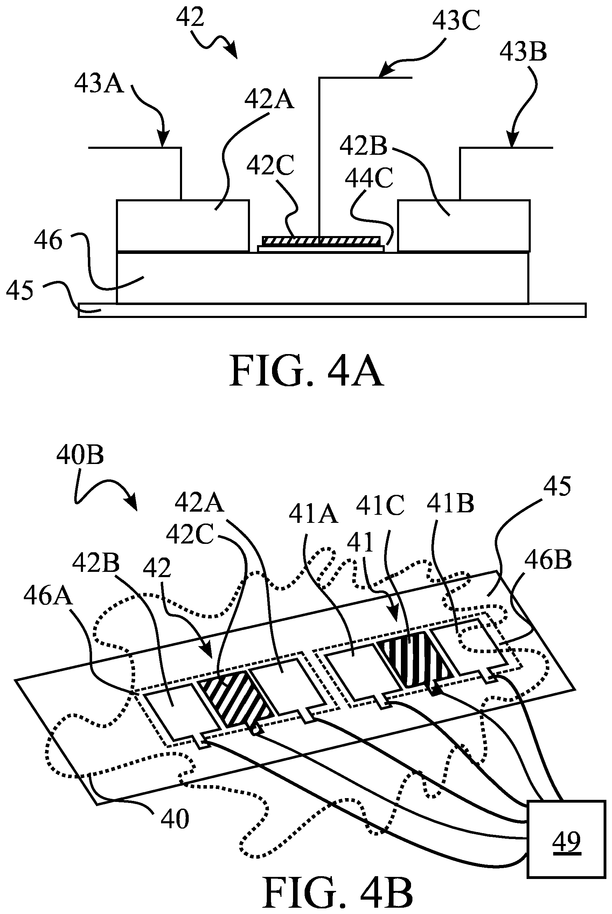

FIG. 4A illustrates a schematic of an electronic device 42 according to an embodiment. The electronic device 42 shown in FIG. 4A, is a field-effect transistor, FET. The FET comprises a source terminal 42A, a drain terminal 42B and a gate terminal 42C. The terminals 42A, 42B and 42C are connected to leads 43A, 43B and 43C, respectively. The gate terminal 42C comprises a composition of MXene and optionally lignin. The source terminal 42A and the drain terminal 42B are connected to one another via a semiconducting material 46. In a further embodiment, said semiconducting material is a semiconductor, such as silicon, and does not comprise MXene. The gate terminal 42C is attached to the semiconducting material 46 via a dielectric 44C. An FET electronic device may be configured to, based on the composition of the gate terminal, provide a gate voltage and/or work function based on the at least one gaseous substance interacting with the gate terminal. In other words, interaction of the at least one gaseous substance with the gate terminal (here, a sensing layer) having a composition of MXene and optionally lignin, may be measured. The electronic device 42 is on a substrate 45.

FIG. 4B illustrates a sensor according to an embodiment and, as a dashed line, at least part of at least one gaseous substance 40. In FIG. 4B, the sensor is an FET sensor. In FIG. 4B, the sensor comprises two electronic devices 41,42 having therein each a source terminal 41A,42A and a drain terminal 41B,42B connected to one another via a semiconductor 46A,46B. Moreover, the electronic devices 41,42 comprise a gate terminal 41C,42C, respectively. The gate terminal 41C, 42C is attached to the semiconductor 46A,46B via a dielectric, such as an oxide layer (not shown in FIG. 4B). The electronic devices 41,42 are on a substrate 45.

In an embodiment, wherein at least one electronic device is a field-effect transistor, the channel of said FET comprises a composition of MXene and optionally lignin. In other words, the channel is the sensing layer of said at least one electronic device. Therefore, the channel may further be arranged to be exposed at least in part to the at least one gaseous substance. In an embodiment, the first electronic device is an FET, wherein the first composition of MXene and optionally lignin is comprised in the channel of the FET. In an embodiment, the second electronic device is an FET, wherein the second composition of MXene and optionally lignin is comprised in the channel of the FET.

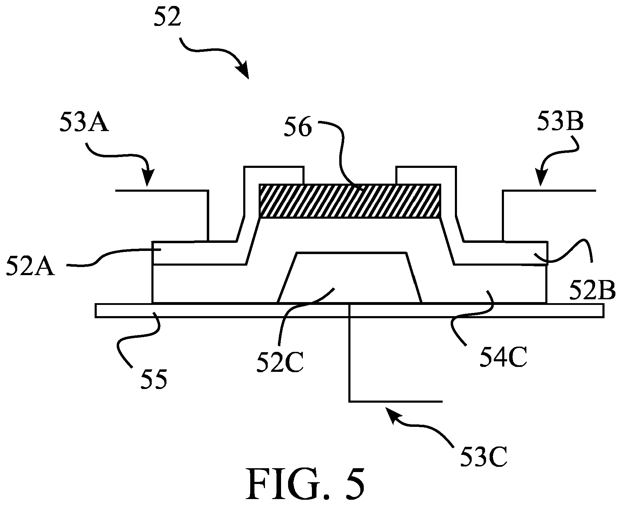

FIG. 5 illustrates a schematic of an electronic device 52 according to an embodiment. The electronic device 52 shown in FIG. 5, is an FET. The FET comprises a channel 56, a source terminal 52A, a drain terminal 52B and a gate terminal 52C similar to the FET electronic device of FIG. 4A. However, in FIG. 5, the source terminal 52A and 52B are attached the channel 56 which comprises a composition of MXene and optionally lignin. In other words, the channel 56 is the sensing layer of the FET electronic device 52. The terminals 52A, 52B and 52C are connected to leads 53A, 53B and 53C, respectively.

Although electronic devices and sensors illustrated in FIG. 2, 3, 4 and 5 illustrate resistive, capacitive and FET electronic devices as separate embodiments, two or more of such designs, or electronic device types, may be comprised in a same sensor according to at least some embodiments. Moreover, in an embodiment, the sensor comprises at least two of: an FET, a capacitor, or a resistor, as electronic devices.

In an embodiment, the plurality of electronic devices further comprises a third electronic device having a third composition of MXene and optionally lignin, said third electronic device having a third sensitivity for electrically sensing the at least one gaseous substance. The third sensitivity is different from the first and second sensitivities of the at least one first and the at least one second electronic devices.

In an embodiment, the sensor comprises a plurality of electronic devices (comprising the at least one first and second electronic devices), said electronic devices having *M* number of different MXenes, *L* number of different chemical compositions of lignin and *N* number of different ratios of the amount of lignin relative to MXene with respect to one another, wherein *M, L* and *N* are positive integers. Therefore, in at least some such embodiments, the sensor comprises *M* × *L* × *N* number of electronic devices having *M* number of different MXenes, *L* number of different chemical compositions of lignin and *N* number of different ratios of the amount of lignin relative to the amount of MXene. Such embodiments may be beneficial at least in that because of plurality of different compositions of electronic devices are arranged to sense the at least one gaseous substance, the differences in sensitivities of said electronic devices, provide a less ill-posed system than what would be obtainable with fewer number of electronic devices, such as one electronic device. The plurality of different electronic devices may also provide a benefit in that such electronic device may better and/or more reliably sense more than one gaseous substance concurrently, for example.

In an embodiment, wherein the sensor comprises *M* × *L* × *N* number of electronic devices, *M* is larger than two. In an embodiment, *L* and/or *N* is at least three. In an embodiment, *M* is 1, *L* is 3 and *N* is 1, and in such an example embodiment the sensor comprises a substrate and three electronic devices having each different chemical composition of lignin. Therefore, the sensor comprises three different electronic devices. For example, a first electronic device of said three electronic devices may comprise a predominantly first type of lignin, such as G units, a second electronic device of said three electronic devices may comprise a predominantly second type of lignin, such as H units, and a third electronic device of said three electronic devices may comprise a predominantly third type of lignin, such as S units. Such first, second and third types are to be understood to mean that such types comprise predominantly of such a unit within manufacturing tolerances of lignin. In other words, other lignin types may be present in composition of an electronic device, but the chemical composition of lignin in the electronic device is such that the mentioned lignin type is a predominant lignin type of the electronic device in question. For example, if a first chemical composition of lignin comprises 80% of H-units, 15% of G-units and 5% of S units (that is, H:G:S ratio of 80:15:5), while a second chemical composition of lignin comprises 60% of S-units, 20% of G-units and 20% of H-units (that is, H:G:S ratio of 20:20:60), then the first chemical composition of lignin may be referred to as a chemical composition having predominantly H-units, and the second chemical composition having predominantly S-units.

In an embodiment, *M* is 1, *L* is 1 and *N* is 2, and in such an embodiment the sensor comprises two different electronic devices having compositions of MXene and lignin having different ratios of the amount of lignin relative to MXene from one another. Therefore, the ratio between MXene and lignin is different between the electronic devices. In other words, a first electronic device of said two electronic devices may comprise a first ratio of the amount of lignin relative to the amount of MXene and a second electronic device of said two electronic devices may comprise a second ratio of the amount of lignin relative to the amount of MXene.

In an embodiment, *M* is 3, *L* is 1 and *N* is 1, and in such an embodiment the sensor comprises three electronic devices having each different MXene. In other words, a first electronic device of said three electronic devices may comprise a first different MXene, a second electronic device of said three electronic devices may comprise a second different MXene, and a third electronic device of said three electronic devices may comprise a third different MXene. The electronic devices of the sensor may be different in terms of MXene in at least one of: MXene structure or MXene type. In an embodiment, *M* is 3, *L* is 3 and *N* is 4, and as such, the sensor comprises 36 different electronic devices in terms of composition of MXene and lignin, and in terms of sensitivity to the at least one gaseous substance. The distribution of MXene of the plurality of electronic devices may be different from one another. In an embodiment, the sensor comprises *D* × *M* × *L* × *N* number of electronic devices having *D* number of different MXene distributions, wherein *D* is a positive non-negative integer. In some embodiments, *D* is 4, *M* is 3, *L* is 3 and *N* is 4, forming thereby 144 electronic devices (4×3×3×4 electronic devices) different in terms of composition of MXene and lignin and different in terms of sensitivity to the at least one gaseous substance. In an embodiment, *D* is at least two. For example, such two different distributions of MXene may be obtained by using two different solvents in the manufacture of the respective electronic devices, such as water, H₂O, and an organic solvent, such as ethanol. In an embodiment, a plurality of different electronic device types *T*, may be comprised in a sensor, wherein T is a positive integer. In an embodiment, the sensor comprises *T* × *D* × *M* × *L* × *N* number of electronic devices, wherein *T* is the number of electronic device types.

One benefit of the plurality of electronic devices, such as more than 5 or more than 10 electronic devices having sensitivities different from one another in terms of composition of MXene and optionally lignin, in accordance with at least some embodiments, is that the differences in sensitivities of such electronic devices of the sensor may be used to sense the at least one gaseous substance thereby providing more information on said gaseous substance than would be obtainable with a single electronic device. Moreover, a plurality of different gaseous substances may be sensed because of differences in the composition in the electronic devices in terms of MXene and optionally lignin and corresponding sensitivities may provide different responses for different gaseous substances. Moreover, because a substrate and electronic devices thereon, provide a simplified structure, both manufacturing and end-of-life, EOL, recycling of such devices and components therein is simple. For example, a resistive electronic device, such as a resistive pattern, may be applied on a substrate in the form of an ink thereby forming a sensing layer according to at least some embodiments. In at least some embodiments, also maintenance requirements of such sensors, such as resistive sensors, may be reduced at least because the number of replacement parts needed is reduced. Moreover, replacing at least part of a sensor is possible without excessive material needs, cost or labor due to a simplified structure of the sensor and parts thereof.

In at least some embodiments, the sensor is part of a measurement system, said system comprising a sensor, and a housing having an attachment portion to a substrate of the sensor. Such embodiments are beneficial in that, the electronic components and devices, such as a computing device, measuring device, transceiver unit or the like, may be comprised in said housing. The sensor may be arranged to attach to, and arranged to detach from, the housing. In at least some embodiments, in a measurement system, the substrate and electronic devices thereon are arranged to be replaceable with another sensor. The sensor may be a pad. The substrate may be arranged to be attached to the housing with an adhesive, such as glue. The substrate may be arranged to be attached to, and detachable from, the housing with a clamping mechanism.

The sensor according to at least some embodiments may be manufactured from suitable precursors providing MXene and optionally lignin. In the following, manufacturing and processing methods for manufacturing a sensor such as those disclosed above, are presented. A sensing layer may be formed from an ink comprising MXene and optionally lignin.

In accordance with the present disclosure, there is provided a method of manufacturing a sensor. A plurality of electronic devices may be formed at least in part from a plurality of corresponding inks having different compositions of MXene and optionally lignin. In some embodiments wherein one or more Ti₃C₂Tₓ types are used as MXene, Ti₃C₂Tₓ may be manufactured as follows. A precursor, such as MAX phase Ti₃AlC₂, is obtained. In order to obtain an MXene of type (that is, Ti₃C₂Tₓ), said precursor may be etched using lithium fluoride, LiF and hydrochloric acid, HCl. Etching may also be done using hydrofluoric acid, HF, and HCl. The etched MXene, Ti₃C₂Tₓ, is then delaminated and washed. Said washing is done so as to achieve a suitable pH. Washing may be done, for example, using purified water, such as distilled water. In some embodiments, a suitable pH for washing is in the range of 5 to 6. Then, the washed and delaminated Ti₃C₂Tₓ is centrifuged, from which well-delaminated Ti₃C₂Tₓ supernatant is collected. Such a well-delaminated Ti₃C₂Tₓ, or other well-delaminated MXene, may be used to obtain at least one ink for at least one electronic device in a sensor according at least some embodiments.

In an embodiment, one or more electronic devices of the sensor comprise compositions of MXene and lignin. For example, one or more sensing layers, such as resistive pattern of a resistor, capacitive pattern of a capacitor, gate terminal of an FET or channel of an FET may comprise composition of MXene and lignin. In an embodiment, at least the first composition comprises MXene and lignin. In an embodiment, a chemical composition of lignin, that is lignin having concentration of S-units, H-units and G-units of lignin, and/or ratios thereof, may be obtained using fractionation. Processing method and solvent utilized may affect the chemical composition of lignin, such as the presence and/or occurrence of S-units, H-units and G-units as well as potential other units of lignin.

In an embodiment, in order to obtain well-dissolved lignin for an ink pattern, lignin raw material is provided. Such lignin raw material may be, for example, "kraft lignin" obtained from kraft processing of wood. Kraft lignin refers to depolymerized and chemically modified lignin isolated from a kraft pulping process. After the provision of lignin raw material, a mixture of lignin and a fractionation solvent is obtained by mixing said lignin raw material with the fractionation solvent. The fractionation solvent may be an organic solvent. Examples of organic solvents include dimethyl sulfoxide (DMSO), N-methylpyrrolidone (NMP), N,N-dimethylacetamide (DMAc), N,N-dimethylformamide (DMF), and ethanol (EtOH). The mixture of lignin and the fractionation solvent is centrifuged, providing a supernatant of well-dissolved lignin. The well-dissolved lignin comprises fractionated lignin suitable for a composition of MXene and lignin. Different chemical compositions of lignin may be obtained using a different fractionation solvent, or a plurality of different fractionation solvents, for example.

In an embodiment, after well-delaminated MXene and well-dissolved lignin are obtained, an ink comprising a composition of solvent, MXene and lignin is manufactured. In some embodiments, the obtained MXene type is Ti₃C₂Tₓ.

In an embodiment, the well-dissolved lignin and the well-delaminated MXene are combined into solution "AB". Solution "AB" is obtained by combining solution "A" comprising the well-dissolved lignin and solution "B" comprising the well-delaminated MXene. Solution "A" may be formed by dissolving the well-dissolved lignin in a fractionation solvent. Such a dissolvement may done in a ratio of well-dissolved lignin to fractionation solvent, for example. In some embodiments, such a ratio is from 1 mg/ml ratio to 5 mg/ml, in terms of ratio of well-dissolved lignin to fractionation solvent. Solution "B" may be formed by dispersing the well-delaminated MXene in water. In order to form solution "B", such dispersion may be done in 1 mg/ml ratio of well-delaminated MXene to that of water, for example.

The formed solution "A" and solution "B" are combined thereby forming a solution "AB". The combination of solution "A" and solution "B", may be done, for example, in a 1:1 volume ratio. Such combination may be enhanced under magnetic stirring, for example. Then, the combined solution "AB" is centrifuged and a sediment is collected. Process of washing and centrifuging of the formed sediment may be done repetitively by removing unreacted MXene and by adding water until the formed supernatant is clear. The final sediment may then be re-dispersed in organic solvent and/or water with a desired concentration, thereby providing an ink comprising a composition of fractionation solvent, MXene and lignin.

In some embodiments, dopant is comprised in the ink, such as the ink obtained from solution "AB". A dopant may be added to the ink, or to solution "AB", for example. In other words, a trace amount of dopant, such as dopant comprising noble metal, metal oxides, and/or functional organic molecules, may be part of, or added into, the ink comprising MXene and optionally lignin, or to a precursor solution thereof, in order to modify selectivity, such as increase the selectivity, of the electronic device ultimately manufactured at least in part from said ink or precursor solution. In other words, such an ink is doped with a dopant.

The provision of ink, such as the examples described above, may then be used to obtain an ink pattern and at least one sensing layer of an electronic device for a sensor, such as at least one resistive pattern for a resistive sensor, at least one electrode for a capacitive sensor, a channel for an FET sensor, and/or a gate terminal for an FET sensor. A sensor in accordance with at least some embodiments may be obtained as follows. A substrate is obtained, and ink is provided on said substrate in order to form an ink pattern on said substrate, and correspondingly a plurality of ink patterns and/or patterns.

In an embodiment, the ink is applied, for example, on a substrate or on a dielectric layer. Examples of such application techniques include printing, writing and pressing. Printing techniques include, for example, inkjet printing, screen printing, roll-to-roll printing, rotogravure printing, extrusion printing, flexography, inkjet printing, and stencil-printing. The ink may be dispensed on a surface. The surface of the first ink pattern and the second ink pattern may be on the substrate, and/or same surface thereof. In some embodiments, an ink having a composition of solvent, MXene and optionally lignin is applied on a substrate so as to form a resistive pattern or a capacitive pattern comprising a corresponding composition of MXene and optionally lignin. In some embodiments, ink having a composition of solvent, MXene and optionally lignin is applied atop a dielectric, such as an oxide layer of a semiconductor of a FET electronic device, for example. In an embodiment, ink is applied so as to form a gate terminal of a FET electronic device, for example. Suitable application techniques for an ink may depend, for example, on the viscosity of said ink and/or the substrate to which such a provision is to be done. Suitable ink composition as well as printing technique used may depend at least on the substrate and specific sensor design and electronic device type(s). After application of an ink to form a pattern, for example, on the substrate, the ink pattern may be dried so as to provide a sensing layer, such as a pattern, having a sensitivity for the at least one gaseous substance which is based on the composition of MXene and optionally lignin therein. Such drying may comprise actively and/or passively removing, such as evaporating, solvent comprised in the ink pattern thereby forming at least part of the corresponding electronic device. In an embodiment wherein a capacitive pattern is formed, one or both of the electrodes is manufactured from a respective ink, wherein one of said inks comprises the composition of MXene and optionally lignin. In an embodiment wherein a capacitive pattern is formed, the two electrodes may be manufactured from different inks, said inks being different from one another in terms of composition of MXene and optionally lignin. One of the electrodes may be manufactured from a conductive ink, such as a metal ink, for example.

A resistor (a resistive electronic device) may be manufactured as follows. An ink having a composition of solvent, MXene and optionally lignin is obtained. The shape of the (resistive) pattern may form a continuous pattern such that the shape is configured to form an electronic circuit from which resistance may be measured. The solvent of an ink composition is removed from the formed pattern thereby forming a resistive pattern.

A capacitor (a capacitive electronic device) may be manufactured as follows. An ink having a composition of solvent, MXene and optionally lignin, and another ink different from said composition, such as an electrically conductive ink, is obtained. The shape of the (capacitive) pattern comprises two separate patterns (i.e., electrodes) separated by a gap, thereby forming the capacitor. The gap may comprise a dielectric material. For a capacitor, one of said patterns (i.e., one of the electrodes) comprises a composition of MXene and optionally lignin. The solvent of the ink composition is removed from the formed ink pattern(s) thereby forming a capacitive pattern. The other electrode may be obtained with a conductive ink. Dielectric material may be provided as a dielectric ink. Dielectric material may be a substrate for the capacitive pattern, having the two electrodes on opposite surfaces of the dielectric material.

Conductive ink (that is, ink that is electrically conductive), such as a metal ink may be obtained as a suspension of a conductive material and a solvent. Such inks may further comprise binder agents, surfactants, or polymers acting as stabilizers. Conductive material may be metal particles or conductive polymers, for example. Conductive material may be a carbon material, such as graphite powder, graphene, and/or carbon nanotube(s). In an embodiment, the conductive ink is non-reactive or substantially inert to the at least one gaseous substance. Dielectric ink(s) may be obtained as a suspension of dielectric material and a solvent. Dielectric material may be a polymer or a co-polymer, such as an UV-curable plastic or a ceramic material, such as silicon dioxide or aluminium oxide. Dielectric ink may be thermally curable siloxane polymer composite, UV cured epoxy and/or polyimide, for example.

An FET electronic device comprising a composition of MXene and optionally lignin as the gate terminal may be manufactured as follows. A semiconducting channel, a source terminal and a drain terminal are obtained using typically available methods known to a person skilled in the art. For example, doping pools for providing source and drain terminals may be provided to the semiconductor material, and said source and drain terminals are formed. Dielectric layer is obtained on a surface of the channel so as to separate the channel from a gate terminal. The dielectric may be deposited using atomic layer deposition, ALD, or printed as an ink comprising a dielectric material, for example. Alternatively, the dielectric layer may be an oxide layer formed on the semiconducting channel. An ink having a composition of solvent, MXene and optionally lignin may be applied onto the dielectric layer on the channel thereby forming a gate terminal of the FET.

An FET electronic device comprising a composition of MXene and lignin as the channel of the FET may be manufactured as follows. In an embodiment, source, gate and drain terminals are printed using electrically conductive ink(s), such as metal inks comprising a metal and a solvent. Dielectric material may be formed on the gate terminal in the form of a dielectric ink comprising a dielectric material. On the dielectric layer, an ink having a composition of solvent, MXene and optionally lignin may be provided thereby forming a channel comprising MXene and optionally lignin. Alternatively, an FET electronic device comprising a composition of MXene and optionally lignin as the channel of the FET may be manufactured as follows. Gate, source and drain terminals may be obtained using typically available FET manufacturing methods. Such manufacturing includes, for example, lithography, etching and/or deposition. On the gate terminal, a dielectric layer is formed. Such dielectric layer may be formed by depositing an oxide layer onto the gate terminal. For example, deposition of a dielectric layer may be done with atomic layer deposition, ALD. A dielectric layer may be applied as a dielectric ink comprising a dielectric material. A channel comprising a composition of solvent, MXene and optionally lignin may then be printed on the dielectric layer. After solvent is removed, a semiconducting channel of the FET electronic device is formed, said semiconducting channel comprising the composition of MXene and optionally lignin.

The methods according to at least some embodiments provide several advantages. For example, manufacturing by providing ink(s), for example on a substrate, provides an easy manufacturing scheme. This is at least because the ink may be provided with, for example, printing writing or pressing. Because an ink according to at least some embodiments is printable, manufacturing is efficient, for example, in terms of production time, material requirements and manufacturing cost. Moreover, a viscous ink may be applied to be part of more complex electronic devices, such as an FET electronic device according to at least some embodiments. Moreover, as the substrate may be a ready-made material, such as paper, the provision of a substrate is simple at least in terms of material costs and preparation time, for example. An advantage according to at least some embodiments is also that a plurality of sensors may be produced on a same substrate, after which such sensors are separated to respective sensors. For example, substrate may be divided into portions by cutting the substrate to respective portions comprising a sensor. Therefore, simple and effective mass production of sensors according to at least some embodiments is possible.

In accordance with the present disclosure, there is provided a method for sensing the at least one gaseous substance using plurality of electronic devices of the sensor. Using electronic devices having different compositions of MXene and optionally lignin, a response is measurable because of an introduction of the at least one gaseous substance to the at least two electronic devices. Such a response may be a resistance value, or change therein, for example. The information on concentration or presence of the at least one gaseous substance is based on the difference in sensitivities of the at least two electronic devices because the composition of MXene and optionally lignin is different between said at least two electronic devices. The at least two electronic devices having different compositions with one another are thus, configured to sense the at least one gaseous substance.

In at least some embodiments, the method further comprises calibrating the sensor by measuring an electrical response, such as resistance or voltage, from known concentrations of the at least one gaseous substance. Such calibration may be beneficial during the initiation of use of the sensor, and/or later on during the life time of the sensor. Such may be the case, for example, if the environment and/or composition of sensing layers and/or electronic devices changes over time. Environmental changes include, for example, changes in temperature and humidity regarding the sensor and surroundings thereof. Calibration, for example because of a change in location of the sensor may also be beneficial.

A calibration may be especially beneficial, for example, directly after printing a plurality of sensors. Such may be beneficial in that, differences in manufacture, such as provision of ink and precision and differences thereof, may be taken into account. Such a calibration may also be done, for example, after the sensor is placed in the environment wherein it is set to operate.

An electrical response, such as resistance or voltage, may be configured to be measured from the plurality of electronic devices comprised in the sensor. Based on the measured electrical response, information on a presence of the at least one gaseous substance may be derived. In an embodiment, the step of deriving information on the presence of at least one gaseous substance comprises determining a type and/or concentration of the at least one gaseous substance. The determined concentration and/or type may be based on a measured electrical response. In at least some embodiments, derivation of information on the presence of at least one gaseous substance comprises determining a type and/or concentration of the at least one gaseous substance.

In an embodiment, an artificial neural network, ANN, is arranged to be used to derive and/or classify information on the presence of the at least one gaseous substance. The ANN model may comprise a plurality of fully connected layers. Moreover, in at least some embodiments, the ANN model comprises nonlinear activation, and feedback optimization. The ANN may thus, provide a benefit in that it may improve the performance of the sensor regarding fault tolerance, selectivity, and recognition accuracy. The lifetime of the sensor array may also be increased by updating the ANN utilized at specific instances, such as after a certain period of time. ANN may be especially beneficial in embodiments, wherein a plurality of different electronic devices are incorporated in a sensor, such as more than 10, 20 or 100 different patterns in terms of composition of MXene and optionally lignin configured to sense the at least one gaseous substance, such as 3, 5 or 10 different gaseous substances.

In an embodiment, the derivation of the information comprises use of an artificial neural network, ANN. In at least some such embodiments, ANN is configured to derive information on the presence of said at least one gaseous substance based on measured (test) electrical response from the plurality of electronic devices. In an embodiment, said ANN comprises an ANN model, and said ANN model is trained using measured test responses from the plurality of electronic devices. The test responses may be measured using a plurality of test samples comprising known concentrations of the at least one gaseous substance. Therefore, the ANN model according to at least some embodiments is adaptive for different tasks, therefore providing a wide area of applications and use cases.

In an embodiment, an ANN model is trained as follows. A plurality of electrical responses measured from the plurality of electronic devices of the sensor when a known concentration of at least one gaseous substance or a combination of gaseous substances is introduced to said sensor and electronic devices therein. Then, electrical response, such as resistance or voltage values, and known concentration pairs are formed wherein the pairs describe the known concentration at which the corresponding electrical response was measured. A plurality of pairs are formed, for example, more than 1000 pairs, or more than 10000 pairs of electrical responses and corresponding concentrations of the at least one gaseous substance. The obtained plurality of pairs may then be divided into a training set, a validation set, and a test set. The division may be, for example 50% of pairs in the training set, 25% of pairs in the validation set and 25% in the test set. In at least some embodiments, the ANN model is trained by the training set with electrical property values and corresponding known concentration of the at least one gaseous substance. During training, the validation set is used to assess the training, thereby preventing overfitting. The trained ANN model may be tested using the test set, that is, using a set of pairs not previously introduced to the ANN model. For an ANN model to be considered to be a trained ANN model, an accuracy of 90% or more with respect to the test set results is expected.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention. Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### EXAMPLE CLAUSES:

Clause 1. A sensor for sensing at least one gaseous substance, the sensor comprising: at least one first electronic device for electrically sensing the at least one gaseous substance with a first sensitivity, said at least one first electronic device comprising a first composition of MXene and optionally lignin; and at least one second electronic device for electrically sensing the at least one gaseous substance with a second sensitivity, said at least one second electronic device comprising a second composition of MXene and optionally lignin; wherein the second composition is different from the first composition such that the second sensitivity differs from the first sensitivity.
Clause 2. The sensor according to clause 1, wherein the at least one first electronic device is a resistor comprising a first resistive pattern comprising the first composition; a capacitor comprising two patterns as electrodes, one of said two electrodes comprising the first composition; a field-effect transistor, FET, having a semiconducting channel comprising the first composition; or a FET having a gate terminal comprising the first composition
Clause 3. The sensor according to any one of clauses 1 or 2, wherein the at least one second electronic device is a resistor comprising a second resistive pattern comprising the second composition; a capacitor comprising two patterns as electrodes, one of said two electrodes comprising the second composition; a field-effect transistor, FET, having a semiconducting channel comprising the second composition; or a FET having a gate terminal comprising the second composition.
Clause 4. The sensor according to any one of clauses 1 to 3, wherein at least the first composition comprises MXene and lignin and/or at least the second composition comprises MXene and lignin.
Clause 5. The sensor according to any one of clauses 1 to 4, wherein the sensor further comprises a substrate having thereon at least the first electronic device, said substrate comprising a biodegradable material, such as a paper-based material or a polylactic acid - polyhydroxy butyrate, PLA-PHB, material.
Clause 6. The sensor according to any one of clauses 1 to 5, wherein the second composition is different from the first composition in terms of at least one of: MXene; lignin; the ratio of the amount of lignin relative to the amount of MXene; or dopant.
Clause 7. The sensor according to clause 6, wherein the second composition is different from the first composition at least in terms of MXene such that the second composition has at least an MXene concentration, a distribution of MXene, an MXene structure, ratio of MXene types and/or an MXene type different from the first composition.
Clause 8. The sensor according to clause 6 or 7, wherein the second composition is different from the first composition at least in terms of lignin such that the second composition has at least a ratio of the amount of lignin to the amount of MXene and/or a chemical composition of lignin different from the first composition.
Clause 9. The sensor according to any one of clauses 1 to 8, wherein the MXene of the first composition and/or the second composition comprises Ti3C2Tx, wherein Ti is titanium, C is carbon and Tx is a functional termination, such as a combination of oxygen O, hydroxyl OH, and fluorine F groups.
Clause 10. The sensor according to any one of clauses 1 to 9, wherein at least the first composition comprises a chemical composition of lignin having predominantly S-units, H-units or G-units.
Clause 11. The sensor according to any one of clauses 1 to 10, wherein the sensor further comprises at least one third electronic device for sensing the at least one gaseous substance with at least one third sensitivity, said at least one third electronic device comprising a third composition of MXene and optionally lignin, and said third composition being different from the first composition and the second composition such that the third sensitivity differs from the first sensitivity and the second sensitivity.
Clause 12. The sensor according to any one of clauses 1 to 11, wherein the sensor comprises more than three electronic devices and each electronic device comprises a composition comprising MXene and optionally lignin for sensing the at least one gaseous substance with a sensitivity, said composition being different from the compositions of the other electronic devices of the sensor such that the sensitivity of each electronic device is different from the sensitivities of the other electronic devices.
Clause 13. The sensor according to clause any one of clauses 1 to 12, wherein sensor comprises M × L × N number of electronic devices having M number of different MXenes, L number of different chemical compositions of lignin and N number of different ratios of the amount of lignin relative to MXene, wherein M, N and L are positive integers and at least one of: N or L is at least two.
Clause 14. The sensor according to clause 13, wherein L and/or N is at least three.
Clause 15. The sensor according to clause 13 or 14, wherein the sensor comprises D×M×L×N number of electronic devices having D number of different MXene distributions, wherein D is at least two.
Clause 16. The sensor according to clause 15, wherein the sensor comprises T×D×M×L×N number of electronic devices, having T number of different electronic device types.
Clause 17. The sensor according to any one of clauses 1 to 16, wherein the first composition is comprised in a first sensing layer of the first electronic device, and said first sensing layer has a thickness from 50 nm, nanometers to 5 µm, micrometers, such as from 0.5 µm to 1 µm, and/or the first sensing layer has a surface area from 0.1 mm² to 1500 mm²; and/or wherein the second composition is comprised in a second sensing layer of the second electronic device, and said first sensing layer has a thickness from 50 nm, nanometers to 5 µm, micrometers, such as from 0.5 µm to 1 µm and/or the second sensing layer has a surface area from 0. 1 mm² to 1500 mm².
Clause 18. The sensor according to any one of clauses 1 to 17, wherein at least the first electronic device and/or second electronic devices comprises a line pattern comprising the composition of MXene and optionally lignin, said line pattern having a width of 10 µm to 1000 µm, or 20 µm to 500 µm, and/or a length of 100 µm to 30 cm, 1 cm to 10 cm, 1 cm to 5 cm, 100 µm to 3000 µm, or 200 µm to 1000 µm.
Clause 19. A method of manufacturing a sensor, the method comprising: providing at least one first electronic device comprising a first composition of MXene and optionally lignin; and providing at least one second electronic device comprising a second composition of MXene and optionally lignin, wherein the second composition is different from the first composition.
Clause 20. The method according to clause 19, wherein at least the first composition comprises MXene and lignin and/or at least the second composition comprises MXene and lignin.
Clause 21. The method according to clause 19 or 20, wherein the providing of the at least one first electronic device comprises: applying at least one first ink pattern comprising a first ink composition comprising first solvent, MXene and optionally lignin; and removing the first solvent, thereby forming the at least one first electronic device, said at least one first electronic device corresponding to the at least one first ink pattern.
Clause 22. The method according to clause 21, wherein the first solvent comprises an organic solvent, such as dimethyl sulfoxide, DMSO, N-methylpyrrolidone, NMP, N,N-dimethylacetamide, DMAc, N,N-dimethylformamide, DMF, or ethanol EtOH.
Clause 23. The method according to any one of clauses 21 or 22, wherein the first solvent comprises water.
Clause 24. The method according to any one of clauses 21 to 23, wherein the at least one first ink pattern is applied on a surface of a substrate, and wherein the at least one first electronic device is a resistor having a form of a pattern comprising the first composition.
Clause 25. The method according to any one of clauses 21 to 23, wherein the at least one first ink pattern is applied on a surface of a substrate, and wherein the at least one first electronic device is a capacitor comprising two electrodes each in a form of a pattern, one of said two electrodes comprising the first composition.
Clause 26. The method according to clause 19 or 20, comprising: applying at least one first ink pattern comprising a first ink composition comprising first solvent, MXene and optionally lignin; and removing the first solvent, thereby forming a gate structure of a field-effect transistor, FET.
Clause 27. The method according to clause 19 or 20, comprising: applying at least one first ink pattern comprising a first ink composition comprising first solvent, MXene and optionally lignin; and removing the first solvent, thereby forming a semiconducting layer for a channel of a field-effect transistor, FET.
Clause 28. The method according to any one of clauses 21 to 27 wherein the applying of the at least one first ink pattern comprises printing, such as extrusion printing, inkjet printing, screen printing, or roll-to-roll printing, the at least one first ink pattern.
Clause 29. The method according to any one of clauses 20 to 28, wherein the at least the first ink composition comprises first solvent, MXene and lignin.
Clause 30. The method according to any one of clauses 19 to 29, wherein the providing of the at least one second electronic device comprises: applying at least one second ink pattern comprising a second ink composition comprising second solvent, MXene and optionally lignin; and removing the second solvent, thereby forming the at least one second electronic device, said at least one second electronic device corresponding to the at least one second ink pattern.
Clause 31. The method according to claim 30, wherein the at least one second ink pattern is applied on a surface of a substrate, and/or wherein the at least one second electronic device is a resistor having a form of a pattern comprising the second composition.
Clause 32. The method according to clause 30, wherein the at least one second ink pattern is applied on a surface of a substrate, and/or wherein the at least one second electronic device is a capacitor comprising two electrodes both in a form of a pattern, one of said two electrodes comprising the second composition.
Clause 33. The method according to any one of clauses 30 to 32, wherein the second solvent comprises an organic solvent, such as at least one of: dimethyl sulfoxide, DMSO, N-methylpyrrolidone, NMP, N,N-dimethylacetamide, DMAc, N,N-dimethylformamide, DMF, or ethanol EtOH.
Clause 34. The method according to any one of clauses 30 to 33, wherein the second solvent comprises water.
Clause 35. The method according to any one of clauses 30 to 34 wherein the applying of the at least one second ink pattern comprises printing said second ink pattern, such as extrusion printing, inkjet printing, screen printing, or roll-to-roll printing, the at least one second ink pattern.
Clause 36. A method for sensing at least one gaseous substance, the method comprising: measuring an electrical response from the electronic devices comprised in the sensor according to any one of clauses 1 to 35; and based on the electrical response, deriving information on a presence of the at least one gaseous substance.
Clause 37. The method according to clause 36, wherein the step of deriving information on the presence of at least one gaseous substance comprises determining a type and/or concentration of the at least one gaseous substance.
Clause 38. The method according to clause 36 or 37, wherein the at least one gaseous substance comprises at least one of: hydrogen H2, ammonia NH3, hydrogen sulfide H2S, nitric oxides NO, NO2, carbon monoxide CO, and volatile organic compound, VOCs, such as, toluene C6H5CH3, ethanol CH3CH2OH, methanol CH3OH, methane CH4, or acetone (CH3)2CO.
Clause 39. The method according to any one of clauses 36 to 38, wherein the deriving of the information comprises using an artificial neural network, ANN, comprising a plurality of fully-connected layers, and wherein said ANN is configured to derive information on the presence of said at least one gaseous substance based on resistance of the patterns; and wherein said ANN comprises an ANN model, said ANN model trained using measured test electrical responses of the electronic devices, said test responses measured using a plurality of test samples comprising known concentrations of the at least one gaseous substance.
Clause 40. The method according to any one of clauses 36 to 39 wherein the method further comprises calibrating the sensor by measuring electrical response from known concentrations of the at least one gaseous substance.

### INDUSTRIAL APPLICABILITY

The embodiments of the present disclosure find industrial applicability in sensing gaseous substances.

### ACRONYMS LIST

| **ACRONYM** | **DEFINTION** | **ACRONYM** | **DEFINTION** |
|---|---|---|---|
| ANN | artificial neural network | DMF | N,N-dimethylformamide |
| EtOH | ethanol | DMSO | dimethyl sulfoxide |
| FET | field-effect transistor | NMP | N-methylpyrrolidone |
| DMAc | N,N-dimethylacetamide | PLA-PHB | polylactic acid - polyhydroxy butyrate |

### REFERENCE SIGNS LIST

| **REFERENCE** | **DEFINITION** | **REFERENCE** | **DEFINITION** |
|---|---|---|---|
| 110, 120, 210, 220, 310, 320, 410, 420, 510, 520, 21,22,23, 24, 31, 32, 41, 42 | electronic device | 31C, 31D 43A, 43B, 43C, 53A, 53B, 53C | lead |
| 105a, 105b, 205a, 305a, 405a, 505a | MXene type | 41A, 42A, 52A | source terminal |
| 406a, 406b, 506a, 506b | lignin type | 41B, 42B, 52B | drain terminal |
| 208a, 208b | MXene structure | 41C, 42C, 52C | gate terminal |
| 20A, 20B, 30C, 40B | sensor | 44C | insulator |
| 21A, 21B, 22A, 22B, 23A, 23B, 24A, 24B | contact area | 46, 56 | channel |
| 25, 35, 45, 55 | substrate | 30 | gaseous substance |
| 31A, 31B, 32A, 32B | electrode | 29, 39, 49 | measurement device |

## Claims

1. A sensor for sensing at least one gaseous substance, the sensor comprising:
- at least one first electronic device for electrically sensing the at least one gaseous substance with a first sensitivity, said at least one first electronic device comprising a first composition of MXene and optionally lignin; and
- at least one second electronic device for electrically sensing the at least one gaseous substance with a second sensitivity, said at least one second electronic device comprising a second composition of MXene and optionally lignin;
wherein the second composition is different from the first composition such that the second sensitivity differs from the first sensitivity.

2. The sensor according to claim 1, wherein the at least one first electronic device is a resistor comprising a first resistive pattern comprising the first composition; a capacitor comprising two patterns as electrodes, one of said two electrodes comprising the first composition; a field-effect transistor, FET, having a semiconducting channel comprising the first composition; or a FET having a gate terminal comprising the first composition

3. The sensor according to any one of claims 1 or 2, wherein the at least one second electronic device is a resistor comprising a second resistive pattern comprising the second composition; a capacitor comprising two patterns as electrodes, one of said two electrodes comprising the second composition; a field-effect transistor, FET, having a semiconducting channel comprising the second composition; or a FET having a gate terminal comprising the second composition.

4. The sensor according to any one of the preceding claims, wherein at least the first composition comprises MXene and lignin.

5. The sensor according to any one of the preceding claims, wherein the second composition is different from the first composition in terms of at least one of: MXene; lignin; the ratio of the amount of lignin relative to the amount of MXene; or dopant.

6. The sensor according to any one of the preceding claims, wherein the sensor further comprises at least one third electronic device for sensing the at least one gaseous substance with at least one third sensitivity, said at least one third electronic device comprising a third composition of MXene and optionally lignin, and said third composition being different from the first composition and the second composition such that the third sensitivity differs from the first sensitivity and the second sensitivity.

7. A method of manufacturing a sensor, the method comprising:
providing at least one first electronic device comprising a first composition of MXene and optionally lignin; and
providing at least one second electronic device comprising a second composition of MXene and optionally lignin, wherein the second composition is different from the first composition.

8. The method according to claim 7, wherein the providing of the at least one first electronic device comprises:
applying at least one first ink pattern comprising a first ink composition comprising first solvent, MXene and optionally lignin;
removing the first solvent, thereby forming the at least one first electronic device,
said at least one first electronic device corresponding to the at least one first ink
pattern; and
wherein the providing of the at least one second electronic device comprises:
applying at least one second ink pattern comprising a second ink composition comprising second solvent, MXene and optionally lignin; and
removing the second solvent, thereby forming the at least one second electronic device, said at least one second electronic device corresponding to the at least one second ink pattern.

9. The method according to claim 8, wherein the at least one first ink pattern is applied on a surface of a substrate, and wherein the at least one first electronic device is a resistor having a form of a pattern comprising the first composition.

10. The method according to claim 8, wherein the at least one first ink pattern is applied on a surface of a substrate, and wherein the at least one first electronic device is a capacitor comprising two electrodes each in a form of a pattern, one of said two electrodes comprising the first composition.

11. The method according to claim 7, comprising:
applying at least one first ink pattern comprising a first ink composition comprising first solvent, MXene and optionally lignin; and
removing the first solvent, thereby forming a gate structure of a field-effect transistor, FET.

12. The method according to claim 7, comprising:
applying at least one first ink pattern comprising a first ink composition comprising first solvent, MXene and optionally lignin; and
removing the first solvent, thereby forming a semiconducting layer for a channel of a field-effect transistor, FET.

13. The method according to any one of claims 8 to 12 wherein the applying comprises printing, such as extrusion printing, inkjet printing, screen printing, or roll-to-roll printing, the at least one first ink pattern.

14. A method for sensing at least one gaseous substance, the method comprising:
measuring an electrical response from the electronic devices comprised in the sensor according to any one of claims 1 to 6; and
based on the electrical response, deriving information on a presence of the at least one gaseous substance.

15. The method according to claim 14, wherein the deriving of the information comprises using an artificial neural network, ANN, comprising a plurality of fully-connected layers, and wherein said ANN is configured to derive information on the presence of said at least one gaseous substance based on resistance of the patterns; and
wherein said ANN comprises an ANN model, said ANN model trained using measured test electrical responses of the electronic devices, said test responses measured using a plurality of test samples comprising known concentrations of the at least one gaseous substance.

## Amended claims

### Amended claims in accordance with Rule 137(2) EPC.

1. A sensor (20A) for sensing at least one gaseous substance, the sensor (20A) comprising:
- at least one first electronic device (21) for electrically sensing the at least one gaseous substance with a first sensitivity, said at least one first electronic device comprising a first composition of MXene and lignin; and
- at least one second electronic device (22) for electrically sensing the at least one gaseous substance with a second sensitivity, said at least one second electronic device (22) comprising a second composition of MXene and lignin;
wherein the second composition is different from the first composition such that the second sensitivity differs from the first sensitivity.

2. The sensor (20A) according to claim 1, wherein the at least one first electronic device (21) is a resistor comprising a first resistive pattern comprising the first composition; a capacitor comprising two patterns as electrodes, one of said two electrodes comprising the first composition; a field-effect transistor, FET, having a semiconducting channel comprising the first composition; or a FET having a gate terminal comprising the first composition

3. The sensor according to any one of claims 1 or 2, wherein the at least one second electronic device is a resistor comprising a second resistive pattern comprising the second composition; a capacitor comprising two patterns as electrodes, one of said two electrodes comprising the second composition; a field-effect transistor, FET, having a semiconducting channel comprising the second composition; or a FET having a gate terminal comprising the second composition.

4. The sensor (20A) according to any one of the preceding claims, wherein the second composition is different from the first composition in terms of at least one of: MXene; lignin; the ratio of the amount of lignin relative to the amount of MXene; or dopant.

5. The sensor (20A) according to claim 4, wherein the second composition is different from the first composition such that the second composition has at least a ratio of the amount of lignin to the amount of MXene and/or a chemical composition of lignin different from the first composition.

6. The sensor (20A) according to any one of the preceding claims, wherein the sensor (20A) further comprises at least one third electronic device (23) for sensing the at least one gaseous substance with at least one third sensitivity, said at least one third electronic device (23) comprising a third composition of MXene and optionally lignin, and said third composition being different from the first composition and the second composition such that the third sensitivity differs from the first sensitivity and the second sensitivity.

7. A method of manufacturing a sensor (20A), the method comprising:
providing at least one first electronic device (21) comprising a first composition of MXene and lignin; and
providing at least one second electronic device (22) comprising a second composition of MXene and lignin, wherein the second composition is different from the first composition.

8. The method according to claim 7, wherein the providing of the at least one first electronic device comprises:
applying at least one first ink pattern comprising a first ink composition comprising first solvent, MXene and lignin;
removing the first solvent, thereby forming the at least one first electronic device (21), said at least one first electronic device (21) corresponding to the at least one first ink pattern; and
wherein the providing of the at least one second electronic device (22) comprises:
applying at least one second ink pattern comprising a second ink composition comprising second solvent, MXene and lignin; and
removing the second solvent, thereby forming the at least one second electronic device (22), said at least one second electronic device corresponding to the at least one second ink pattern.

9. The method according to claim 8, wherein the at least one first ink pattern is applied on a surface of a substrate (25), and wherein the at least one first electronic device (21) is a resistor having a form of a pattern comprising the first composition.

10. The method according to claim 8, wherein the at least one first ink pattern is applied on a surface of a substrate (25), and wherein the at least one first electronic device (21) is a capacitor comprising two electrodes each in a form of a pattern, one of said two electrodes comprising the first composition.

11. The method according to claim 7, comprising:
applying at least one first ink pattern comprising a first ink composition comprising first solvent, MXene and lignin; and
removing the first solvent, thereby forming a gate structure of a field-effect transistor, FET.

12. The method according to claim 7, comprising:
applying at least one first ink pattern comprising a first ink composition comprising first solvent, MXene and lignin; and
removing the first solvent, thereby forming a semiconducting layer for a channel of a field-effect transistor, FET.

13. The method according to any one of claims 8 to 12 wherein the applying comprises printing, such as extrusion printing, inkjet printing, screen printing, or roll-to-roll printing, the at least one first ink pattern.

14. A method for sensing at least one gaseous substance, the method comprising:
measuring an electrical response from the electronic devices (21,22) comprised in the sensor (20A) according to any one of claims 1 to 6; and
based on the electrical response, deriving information on a presence of the at least one gaseous substance.

15. The method according to claim 14, wherein the deriving of the information comprises using an artificial neural network, ANN, comprising a plurality of fully-connected layers, and wherein said ANN is configured to derive information on the presence of said at least one gaseous substance based on resistance of the patterns; and
wherein said ANN comprises an ANN model, said ANN model trained using measured test electrical responses of the electronic devices, said test responses measured using a plurality of test samples comprising known concentrations of the at least one gaseous substance.
